# EUROPEAN PATENT SPECIFICATION

(11) **EP 4 188 219 B1**
(45) Date of publication and mention of the grant of the patent: **01.01.2025**
(21) Application number: 21778282.0
(22) Date of filing: 30.08.2021
(51) Int. Cl.: A61B 5/15, A61B 5/153, A61M 5/31

(54) **ASPIRATION SYRINGES**
ASPIRATIONSSPRITZEN
SERINGUES D'ASPIRATION

(30) Priority: 31.08.2020 US 202063072711 P
(43) Date of publication of application: 07.06.2023
(73) Proprietor: Bard Access Systems, Inc., Salt Lake City, UT 84116 (US)
(72) Inventor: THORNLEY, Kyle, G., Farmington, UT 84025 (US); STONE, Bryan, Fruit Heights, UT 84037 (US)
(74) Representative: Hoffmann Eitle
(86) International application number: PCT/US2021/048275
(87) International publication number: WO 2022/047326

(56) References cited:
- WO-A1-03/068073
- WO-A1-2020/113123
- DE-U1- 202012 006 191
- US-A- 5 115 816
- US-A1- 2005 215 956

## Description

### BACKGROUND

Traditional syringes were primarily designed for infusion of fluids. However, such syringes are commonly used to draw a slight vacuum during blood-vessel cannulation. The vacuum provides a clinician a means for receiving tactile feedback upon successfully establishing blood-vessel access, the feedback resulting from a pressure differential upon release of the vacuum when a tip of a needle coupled to the syringe enters the blood vessel. With traditional syringes, there is a lack of support for the clinician to both secure the syringe and draw the vacuum while inserting the needle into the blood vessel. What is needed is an aspiration syringe that is specifically configured with at least the foregoing support for blood-vessel cannulation.
DE 20 2012 006191 U1 relates to a medical syringe with a syringe cylinder, which is designed as a cylindrical cavity open on one side, and with a piston that can be moved in the axial direction and seals tightly against the inner wall of the cylinder, which is provided with a piston rod that leads to the open end of the syringe cylinder and that it has a plunger handle at the free end and a finger rest projecting laterally at the open end of the syringe barrel.
WO 2020/113123 A1 relates to syringe accessory devices, specifically to those devices which provide the user an ergonomic advantage during aspiration.

Disclosed herein are aspiration syringes and non claimed methods thereof

### SUMMARY

According to the present invention there is provided an aspiration syringe according to claim 1 comprising a barrel, a plunger disposed in the barrel, and an aspiration mechanism. The aspiration mechanism includes a thumb-support member coupled to a proximal portion of the barrel and a syringe housing slidably disposed around the barrel. The syringe housing includes a proximal portion coupled to a proximal portion of the plunger and a distal portion terminating with a flange incorporated into a finger-support member. The aspiration mechanism is configured for withdrawing the plunger from the barrel as the finger-support member is slid over the barrel toward the thumb-support member. Preferred embodiments of the present invention are defined in the dependent claims.

In some embodiments, the syringe housing and the plunger are molded together in an integral piece such that the proximal portion of the syringe housing is integral with the proximal portion of the plunger.

In some embodiments, the syringe housing and the plunger are separately molded pieces coupled together in a coupled piece such that the proximal portion of the syringe housing is bonded or adhered to the proximal portion of the plunger.

In some embodiments, the syringe housing includes a longitudinal gap within which the thumb-support member moves relative to the syringe housing when the syringe housing is slid over the barrel.

In some embodiments, the flange includes a pair of finger recesses in a distal face of the flange. The pair of finger recesses is configured to respectively cradle a pair of finger pads of a pair of fingers of the single hand. The pair of finger recesses is also configured to encourage placement of each finger of the pair of fingers on an opposite side of the barrel.

In some embodiments, the flange is annular such that the flange encircles the barrel. Alternatively, the flange is coupled to a distal annular member with the distal annular member encircling the barrel. The flange or the distal annular member is configured to accommodate a pair of finger pads of a pair of fingers of the single hand placed against a distal side thereof in any chosen placement of each finger of the pair of fingers around the flange or the distal annular member.

In some embodiments, the flange or the distal annular member includes an annular finger recess in a distal face thereof. The annular finger recess is configured to cradle the pair of finger pads of the pair of fingers.

In some embodiments, the thumb-support member includes a single thumb recess in a proximal face of the thumb-support member. The thumb recess is configured to cradle a thumbpad of a thumb. The thumb recess is also configured to encourage approximately longitudinal alignment of the thumb with a surface normal of a surface of the barrel.

In some embodiments, the thumb-support member is coupled to or formed with a proximal annular member with the proximal annular member encircling the barrel. The proximal annular member is configured to accommodate a thumbpad of a thumb placed against a proximal side thereof in any chosen placement of the thumb around the proximal annular member.

In some embodiments, the proximal annular member includes an annular thumb recess in a proximal face thereof. The annular thumb recess is configured to cradle the thumbpad of the thumb.

In some embodiments, a distal portion of the barrel terminates with a Luer-tapered syringe tip.

In some embodiments, the distal portion of the barrel further terminates with an internally threaded connector around the syringe tip. The internally threaded connector is configured to screw together with an externally threaded or flanged connector of another medical device.

Also disclosed herein is a non claimed method of cannulating with an aspiration syringe. The method includes an obtaining step, a needle-inserting step, and a vacuum-drawing step. The obtaining step includes obtaining the aspiration syringe. The aspiration syringe includes a thumb-support member coupled to a proximal portion of a barrel and a syringe housing slidably disposed around the barrel. The syringe housing includes a proximal portion coupled to a proximal portion of a plunger, as well as a distal portion terminating with a flange incorporated into a finger-support member. The needle-inserting step includes inserting a needle coupled to the aspiration syringe into subcutaneous tissue of a patient. The vacuum-drawing step includes drawing a vacuum with the aspiration syringe while inserting the needle into the subcutaneous tissue of the patient. The vacuum-drawing step is performed with a single hand over a medial portion of the aspiration syringe by squeezing together the finger-support member and the thumb-support member. Squeezing together the finger-support member and the thumb-support member proximally slides the syringe housing over the barrel and withdraws the plunger from the barrel.

In some embodiments, the method further includes a needle-coupling step. The needle-coupling step includes coupling a needle to the aspiration syringe, which, in turn, includes screwing a flanged connector of a needle hub into an internally threaded connector around a syringe tip of the aspiration syringe.

In some embodiments, the squeezing together of the finger-support member and the thumb-support member during the vacuum-drawing step includes moving the thumb-support member through a longitudinal gap within the syringe housing.

In some embodiments, the method further includes a finger-placing step. The finger-placing step includes placing a pair of finger pads of a pair of fingers of the single hand into a pair of finger recesses in a distal face of the flange on opposite sides of the barrel. The finger-placing step is performed before the vacuum-drawing step.

In some embodiments, an alternative of the finger-placing step includes placing the pair of finger pads of the pair of fingers of the single hand anywhere against a distal side of the flange when the flange is annular. When the flange is coupled to a distal annular member, the finger-placing step includes placing the pair of finger pads of the pair of fingers of the single hand anywhere against a distal side of the distal annular member. Again, the finger-placing step is performed before the vacuum-drawing step.

In some embodiments, the alternative of the finger-placing step includes placing the pair of finger pads in an annular finger recess in a distal face of the flange or the distal annular member.

In some embodiments, the method further includes a thumb-placing step. The thumb-placing step includes placing a thumbpad of a thumb of the single hand into a single thumb recess in a proximal face of the thumb-support member with the thumb approximately longitudinally aligned with a surface normal of a surface of the barrel. The finger-placing step is performed before the vacuum-drawing step.

In some embodiments, an alternative of the thumb-placing step includes placing the thumbpad of the thumb of the single hand anywhere against a proximal side of a proximal annular member coupled to or formed with the thumb-support member. Again, the thumb-placing step is performed before the vacuum-drawing step.

In some embodiments, the alternative of the thumb-placing step includes placing the thumbpad in an annular thumb recess in a proximal face of the proximal annular member.

In some embodiments, the method further includes a needle tract-establishing step. The needle tract-establishing step includes establishing a needle tract from a skin surface of the patient to a fluid-containing space within the patient. Establishment of the needle tract is realized upon receiving tactile feedback resulting from a pressure differential occurring with a release of the vacuum when a tip of the needle enters the fluid-containing space.

In some embodiments, the fluid-containing space is a blood-vessel lumen including blood as a fluid therein.

Also disclosed herein is a non claimed method of aspirating a fluid with an aspiration syringe. The method includes an obtaining step, a needle tract-establishing step, and a fluid-aspirating step. The obtaining step includes obtaining the aspiration syringe. The aspiration syringe includes a thumb-support member coupled to a proximal portion of a barrel and a syringe housing slidably disposed around the barrel. The syringe housing includes a proximal portion coupled to a proximal portion of a plunger, as well as a distal portion terminating with a flange incorporated into a finger-support member. The needle tract-establishing step includes establishing a needle tract with a needle coupled to the aspiration syringe from a skin surface to a fluid-containing space within a patient. The fluid-aspirating step includes aspirating fluid from the fluid-containing space with a single hand over a medial portion of the aspiration syringe by squeezing together the finger-support member and the thumb-support member. Squeezing together the finger-support member and the thumb-support member proximally slides the syringe housing over the barrel and withdraws the plunger from the barrel.

In some embodiments, the method further includes a needle-coupling step. The needle-coupling step includes coupling a needle to the aspiration syringe, which, in turn, includes screwing a flanged connector of a needle hub into an internally threaded connector around a syringe tip of the aspiration syringe.

In some embodiments, the squeezing together of the finger-support member and the thumb-support member during the fluid-aspirating step includes moving the thumb-support member through a longitudinal gap within the syringe housing.

In some embodiments, the method further includes a finger-placing step. The finger-placing step includes placing a pair of finger pads of a pair of fingers of the single hand into a pair of finger recesses in a distal face of the flange on opposite sides of the barrel. The finger-placing step is performed before the fluid-aspirating step.

In some embodiments, an alternative of the finger-placing step includes placing the pair of finger pads of the pair of fingers of the single hand anywhere against a distal side of the flange when the flange is annular. When the flange is coupled to a distal annular member, the finger-placing step includes placing the pair of finger pads of the pair of fingers of the single hand anywhere against a distal side of the distal annular member. Again, the finger-placing step is performed before the fluid-aspirating step.

In some embodiments, the alternative of the finger-placing step includes placing the pair of finger pads in an annular finger recess in a distal face of the flange or the distal annular member.

In some embodiments, the method further includes a thumb-placing step. The thumb-placing step includes placing a thumbpad of a thumb of the single hand into a single thumb recess in a proximal face of the thumb-support member with the thumb approximately longitudinally aligned with a surface normal of a surface of the barrel. The finger-placing step is performed before the fluid-aspirating step.

In some embodiments, an alternative of the thumb-placing step includes placing the thumbpad of the thumb of the single hand anywhere against a proximal side of a proximal annular member coupled to or formed with the thumb-support member. Again, the thumb-placing step is performed before the fluid-aspirating step.

In some embodiments, the alternative of the thumb-placing step includes placing the thumbpad in an annular thumb recess in a proximal face of the proximal annular member.

In some embodiments, the fluid-containing space is a blood-vessel lumen and the fluid is blood.

These and other features of the concepts provided herein will become more apparent to those of skill in the art in view of the accompanying drawings and following description, which describe particular embodiments of such concepts in greater detail.

### DRAWINGS

FIG. 1 illustrates a perspective view of an aspiration syringe in a ready-to-aspirate state in accordance with some embodiments.
FIG. 2 illustrates a perspective view of the aspiration syringe after aspiration of a volume of a fluid in accordance with some embodiments.
FIG. 3 illustrates a top view of the aspiration syringe of FIG. 1 in accordance with some embodiments.
FIG. 4 illustrates a top view of the aspiration syringe of FIG. 2 in accordance with some embodiments.
FIG. 5 illustrates a perspective view of another aspiration syringe in a ready-to-aspirate state in accordance with some embodiments.
FIG. 6 illustrates a perspective view of the other aspiration syringe after aspiration of a volume of a fluid in accordance with some embodiments.
FIG. 7 illustrates a top view of the aspiration syringe of FIG. 5 in accordance with some embodiments.
FIG. 8 illustrates a top view of the aspiration syringe of FIG. 6 in accordance with some embodiments.

### DESCRIPTION

It should be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

As set forth above, an aspiration syringe is needed that is specifically configured for a clinician to both secure the aspiration syringe and draw a vacuum during blood-vessel cannulation for receiving tactile feedback upon successfully establishing blood-vessel access. Disclosed herein are aspiration syringes and non claimed methods thereof.

### Aspiration syringes

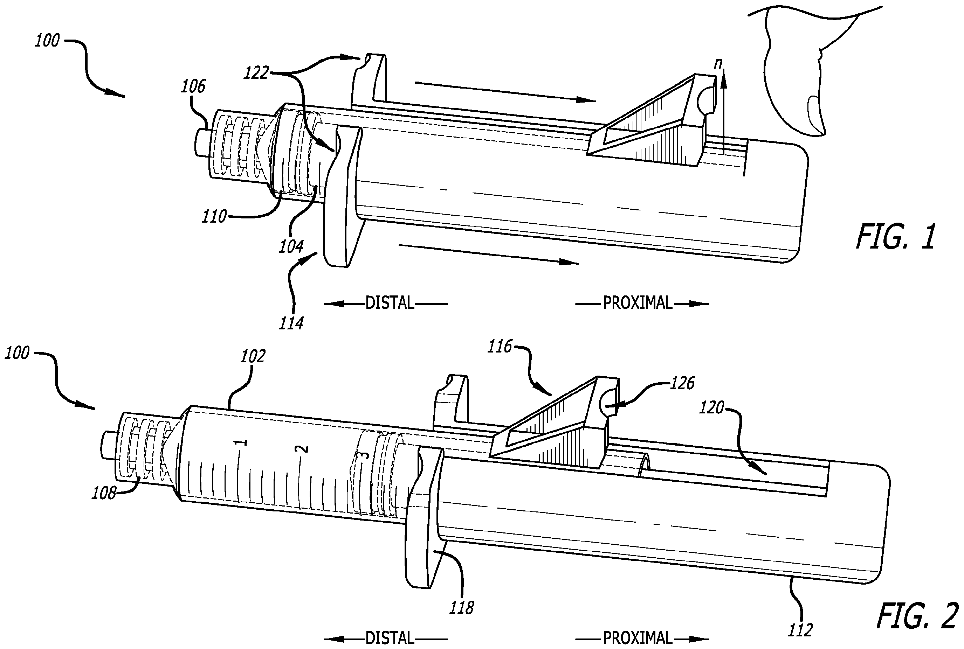

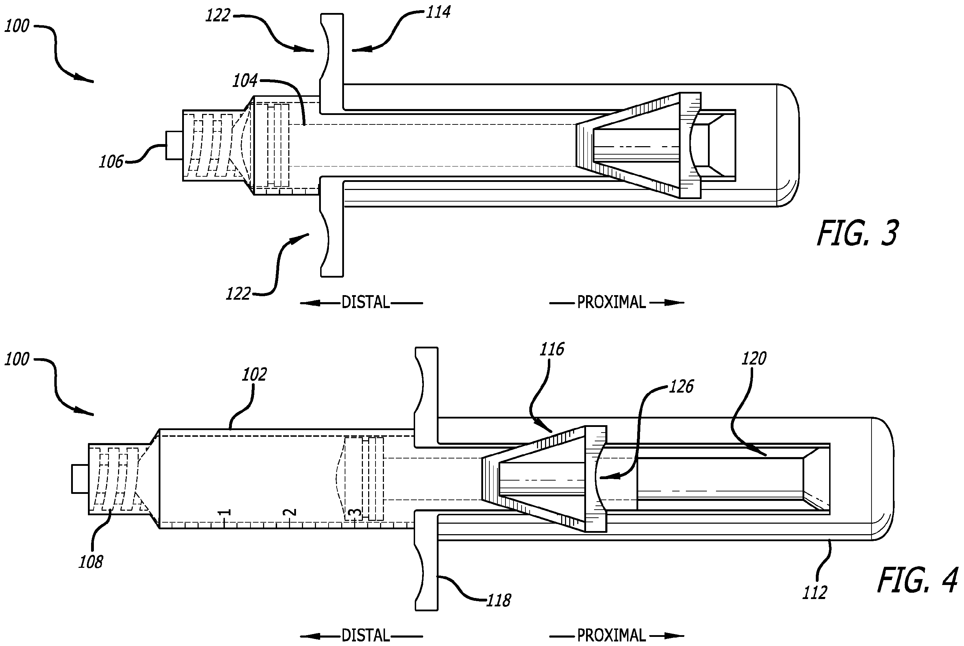

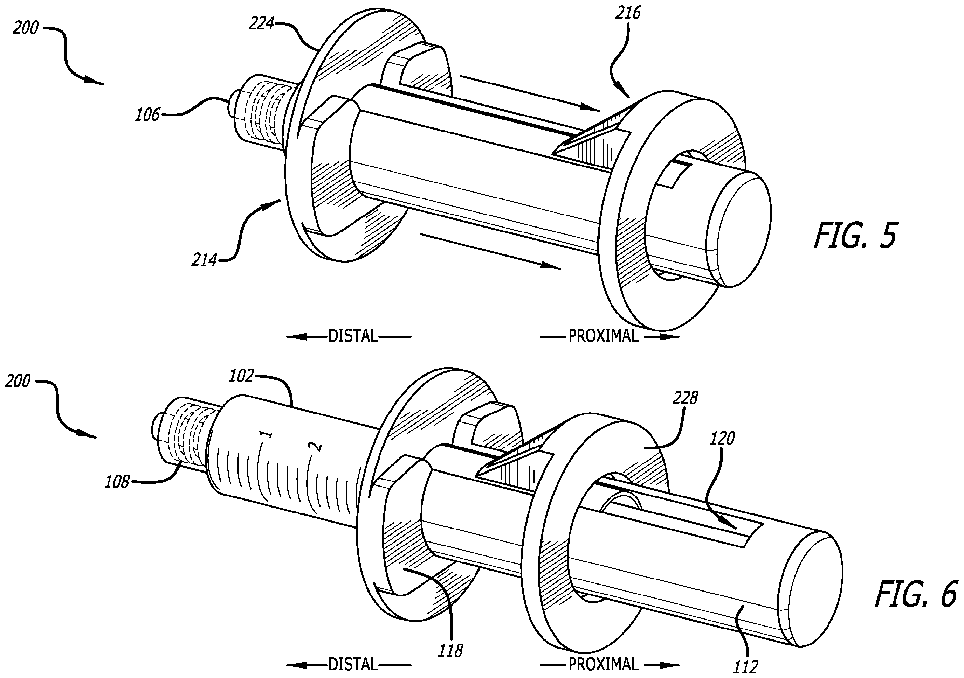

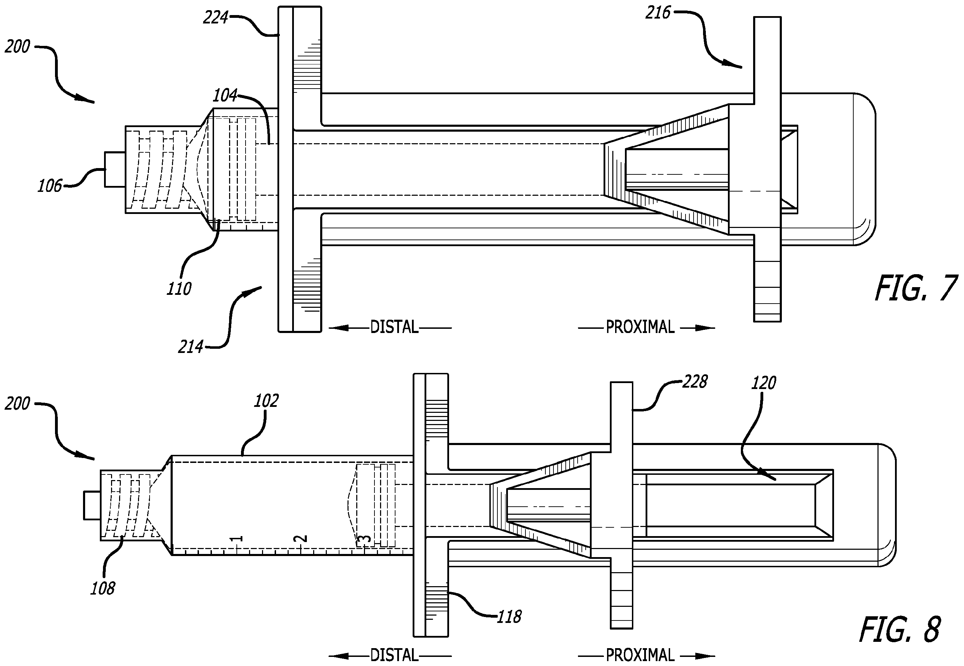

FIGS. 1-4 and FIGS. 5-8 illustrate two different views and two different states for two aspiration syringes in accordance with some embodiments. FIGS. 1 and 3 illustrate an aspiration syringe 100 in a ready-to-aspirate state, while FIGS. 2 and 4 illustrate the aspiration syringe 100 after aspiration of a volume of a fluid. Likewise, FIGS. 5 and 7 illustrate an aspiration syringe 200 in a ready-to-aspirate state, while FIGS. 6 and 8 illustrate the aspiration syringe 200 after aspiration of a volume of a fluid. In FIGS. 2, 4, 6, and 8 the fluid is merely air; however, the fluid can be a bodily fluid such as blood as set forth in the non claimed methods below with respect to at least the fluid-aspirating step.

As shown, the aspiration syringe 100 or 200 includes a barrel 102, a plunger 104 disposed in the barrel 102, and an aspiration mechanism.

The barrel 102 includes a proximal portion terminating with a proximal opening (not shown) through which the plunger 104 is disposed. The barrel 102 also includes a distal portion terminating with a Luer-tapered syringe tip 106 having a distal opening, the syringe tip 106 configured to insert into a Luer-tapered receptacle of another medical device (e.g., a needle including a needle hub having the receptacle). The distal portion of the barrel 102 further terminates with an internally threaded connector 108 (e.g., a collar) around the syringe tip 106. The internally threaded connector 108 is configured to screw together with an externally threaded or flanged connector of the other medical device (e.g., the needle including the needle hub having the flanged connector).

The barrel 102 further includes graduations printed or molded onto the barrel 102 for determining how much fluid is aspirated into the aspiration syringe 100 or 200 when the plunger 104 is withdrawn from its initial position in the barrel 102 in the ready-to-aspirate state of the aspiration syringe 100 or 200.

The plunger 104 includes a proximal portion fixedly coupled to the syringe housing 112 as set forth below with respect to the syringe housing 112. The plunger 104 also includes a distal portion terminating with a gasket 110, the gasket 110 configured to fluidly seal off adjoining portions of the barrel 102 from each other, particularly upon aspiration of a volume of a fluid with the aspiration syringe 100 or 200.

The aspiration mechanism includes a syringe housing 112 slidably disposed around the barrel 102, a finger-support member 114 or 214, and a thumb-support member 116 or 216. The aspiration mechanism is configured for withdrawing the plunger 104 from the barrel 102 as the syringe housing 112 is slid over the barrel 102 and the finger-support member 114 or 214 is brought toward the thumb-support member 116 or 216 such as by squeezing the finger-support member 114 or 214 and thumb-support member 116 or 216 together.

The syringe housing 112 includes a proximal portion fixedly coupled to a proximal portion of the plunger 104 and a distal portion terminating with a flange 118 incorporated into the finger-support member 114 or 214. The syringe housing 112 and the plunger 104 can be separately molded pieces coupled together in a coupled piece such that the proximal portion of the syringe housing 112 is adhered or bonded (e.g., solvent bonded) to the proximal portion of the plunger 104. Alternatively, the syringe housing 112 and the plunger 104 can be molded together in an integral piece such that the proximal portion of the syringe housing 112 is integral with the proximal portion of the plunger 104.

The syringe housing 112 includes a longitudinal gap 120 within which the thumb-support member 116 or 216 moves when the syringe housing 112 is slid over the barrel 102 (e.g., by squeezing the finger-support member 114 or 214 and thumb-support member 116 or 216 together). As set forth below, the thumb-support member 116 or 216 is fixedly coupled to the barrel 102, which restricts the thumb-support member 116 or 216 from moving. Indeed, it is the syringe housing 112 that moves as it is proximally slid over the barrel 102. When the syringe housing 112 is the frame of reference, however, the thumb-support member 116 or 216 moves relative to the syringe housing 112 in the longitudinal gap 120.

The finger-support member 114 or 214 incorporates the flange 118 of the syringe housing 112. Description for each finger-support member of the finger-support members 114 and 214 is set forth below. It should be understood that while the finger-support member 114 is shown to be part of the aspiration syringe 100 shown in FIGS. 1-4, the finger-support member 114 can alternatively be part of the aspiration syringe 200 shown in FIGS. 5-8. Likewise, while the finger-support member 214 is shown to be part of the aspiration syringe 200 shown in FIGS. 5-8, the finger-support member 214 can alternatively be part of the aspiration syringe 100 shown in FIGS. 1-4.

The finger-support member 114 includes a pair of finger recesses 122 in a distal face of the flange 118. The pair of finger recesses 122 is configured to respectively cradle a pair of finger pads of a pair of fingers of a single hand. The pair of finger recesses 122 is also configured to encourage placement of each finger of the pair of fingers on an opposite side of the barrel 102.

The finger-support member 214 is a distal annular member 224 fixedly coupled to the flange 118. Alternatively, the finger-support member 214 is formed together with the flange 118 when the flange 118 is annular. Regardless, the distal annular member 224 or the flange 118, when annular, encircles the barrel 102 such that the barrel 102 passes through a central hole of the distal annular member 224 or the flange 118. When the distal annular member 224 is coupled to the flange 118, the distal annular member 224 and the flange 118 can be separately molded pieces coupled (e.g., adhered, solvent bonded, etc.) together in a coupled piece. Alternatively, the distal annular member 224 and the flange 118 can be molded together in an integral piece. Like the finger-support member 114, the distal annular member 224 or the flange 118, when annular, is configured to accommodate a pair of finger pads of a pair of fingers of a single hand placed against a distal side thereof; however, the distal annular member 224 or the flange 118 is configured to encourage placement of the pair of finger against the distal side of the distal annular member 224 or the flange 118 in any desired placement of the pair of fingers around the distal annular member 224 or the flange 118. While not shown, the distal annular member 224 can include an annular finger recess in a distal face thereof. The annular finger recess is configured to cradle the pair of finger pads of the pair of fingers.

The thumb-support member 116 or 216 is fixedly coupled (e.g., adhered, solvent bonded, etc.) to and extends from the proximal portion of the barrel 102. Description for each thumb-support member of the thumb-support members 116 and 216 is set forth below. It should be understood that while the thumb-support member 116 is shown to be part of the aspiration syringe 100 shown in FIGS. 1-4, the thumb-support member 100 can alternatively be part of the aspiration syringe 200 shown in FIGS. 5-8. Likewise, while the thumb-support member 216 is shown to be part of the aspiration syringe 200 shown in FIGS. 5-8, the thumb-support member 216 can alternatively be part of the aspiration syringe 100 shown in FIGS. 1-4.

The thumb-support member 116 includes a single thumb recess 126 in a proximal face of the thumb-support member 116. The thumb recess 126 is configured to cradle a thumbpad of a thumb of a same hand as the pair of fingers used on the finger-support member 114 or 214 for three-finger aspiration of the aspiration syringe 100 with a natural grip of the hand. The thumb recess 126 is also configured to encourage approximately longitudinal alignment of the thumb with a surface normal of a surface of the barrel 102 as shown in FIG. 1.

The thumb-support member 216 is a proximal annular member 228 fixedly coupled to the thumb-support member 116. Alternatively, the thumb-support member 216 is formed together with the proximal annular member 228 in that merely the proximal annular member 228 is fixedly coupled to the proximal portion of the barrel 102. Regardless, the proximal annular member 228 encircles the barrel 102 such that the barrel 102 passes through a central hole of the proximal annular member 228. Indeed, the thumb-support member 216 and the proximal annular member 228 can be separately molded pieces coupled (e.g., adhered, solvent bonded, etc.) together in a coupled piece. Alternatively, the thumb-support member 216 and the proximal annular member 228 can be molded together in an integral piece. Like the thumb-support member 116, the proximal annular member 228 is configured to accommodate a thumbpad of a thumb of a same hand as the pair of fingers used on the finger-support member 114 or 214 placed against a proximal side thereof; however, the proximal annular member 228 is configured to encourage placement of the thumb against the proximal side of the proximal annular member 228 in any desired placement of the thumb around the proximal annular member 228 for three-finger aspiration of the aspiration syringe 200 with a natural grip of the hand. While not shown, the proximal annular member 228 can include an annular thumb recess in a proximal face thereof. The annular thumb recess is configured to cradle the thumbpad of the thumb. Non claimed methods

Methods of the aspiration syringes 100 and 200 include methods of cannulating, as well as methods of aspirating with the aspiration syringes 100 and 200. Being as cannulation of, for example, a fluid-containing space such as a blood-vessel lumen occurs before aspiration of fluid such as blood therefrom, a method of cannulating with the aspiration syringe 100 or 200 is set forth below including steps for aspirating with the aspiration syringe 100 or 200.

The method of cannulating with the aspiration syringe 100 or 200 includes an obtaining step, a needle-inserting step, and a vacuum-drawing step.

The obtaining step includes obtaining the aspiration syringe 100 or 200. As set forth above, the aspiration syringe 100 or 200 includes the thumb-support member 116 or 216 coupled to the distal portion of the barrel 102 and the syringe housing 112 slidably disposed around the barrel 102. The syringe housing 112 includes the proximal portion coupled to the proximal portion of the plunger 104, as well as the distal portion terminating with the flange 118 incorporated into the finger-support member 114 or 214.

Before the needle-inserting step, the method can further include a needle-coupling step. The needle-coupling step includes coupling a needle to the aspiration syringe 100 or 200, which, in turn, includes screwing a flanged connector of a needle hub into the internally threaded connector 108 around the syringe tip 106 of the aspiration syringe 100 or 200.

The needle-inserting step includes inserting the needle coupled to the aspiration syringe 100 or 200 into subcutaneous tissue of a patient.

Before the vacuum-drawing step, the method further includes a finger-placing step. With respect to the aspiration syringe 100, the finger-placing step includes placing a pair of finger pads of a pair of fingers of the single hand into the pair of finger recesses 122 in the distal face of the flange 118 on opposite sides of the barrel 102. When aspirating with the aspiration syringe 100 as set forth below, the finger-placing step is performed before the fluid-aspirating step if the single hand is partially or wholly removed from the aspiration syringe 100 after any intervening steps between cannulating and aspirating with the aspiration syringe 100.

An alternative of the foregoing finger-placing step is performed for the aspiration syringe 200 or any like syringe including an annular flange or the distal annular member 224. Such an alternative finger-placing step includes placing the pair of finger pads of the pair of fingers of the single hand anywhere against the distal side of the flange 118 when the flange 118 is annular. When the flange 118 is coupled to the distal annular member 224, the finger-placing step includes placing the pair of finger pads of the pair of fingers of the single hand anywhere against the distal side of the distal annular member 224. For example, the alternative of the finger-placing step can include placing the pair of finger pads in the annular finger recess in the distal face of the flange 118 or the distal annular member 224. Like the foregoing finger-placing step, the alternative finger-placing step is performed before the vacuum-drawing step. When aspirating with the syringe 200 as set forth below, the alternative finger-placing step is performed before the fluid-aspirating step if the single hand is partially or wholly removed from the aspiration syringe 200 after any intervening steps between cannulating and aspirating with the aspiration syringe 200.

Further before the vacuum-drawing step, the method further includes a thumb-placing step. With respect to the aspiration syringe 100, the thumb-placing step includes placing a thumbpad of a thumb of the single hand into the single thumb recess 126 in the proximal face of the thumb-support member 116 with the thumb approximately longitudinally aligned with a surface normal of the surface of the barrel 102. When aspirating with the aspiration syringe 100 as set forth below, the finger-placing step is performed before the fluid-aspirating step if the single hand is partially or wholly removed from the aspiration syringe 100 after any intervening steps between cannulating and aspirating with the aspiration syringe 100.

An alternative of the foregoing thumb-placing step is performed for the aspiration syringe 200 or any like syringe including the proximal annular member 228. Such an alternative thumb-placing step includes placing the thumbpad of the thumb of the single hand anywhere against the proximal side of the proximal annular member 228 coupled to or formed with the thumb-support member 216. For example, the alternative of the thumb-placing step can include placing the thumbpad in the annular thumb recess in the proximal face of the proximal annular member 228. Like the foregoing thumb-placing step, the alternative thumb-placing step is performed before the vacuum-drawing step. When aspirating with the syringe 200 as set forth below, the alternative thumb-placing step is performed before the fluid-aspirating step if the single hand is partially or wholly removed from the aspiration syringe 200 after any intervening steps between cannulating and aspirating with the aspiration syringe 200.

The vacuum-drawing step includes drawing a vacuum with the aspiration syringe 100 or 200 while inserting the needle into the subcutaneous tissue of the patient such as while actively performing the needle-inserting step or pausing somewhere in a middle of the needle-inserting step to perform the vacuum-drawing step. The vacuum-drawing step is performed with the single hand over, under, or to a side of a medial portion of the aspiration syringe 100 or 200 by squeezing together the finger-support member 114 or 214 and the thumb-support member 116 or 216. Squeezing together the finger-support member 114 or 214 and the thumb-support member 116 or 216 proximally slides the syringe housing 112 over the barrel 102 and withdraws the plunger 104 from the barrel 102 creating the vacuum. The squeezing together of the finger-support member 114 or 214 and the thumb-support member 116 or 216 during the vacuum-drawing step includes moving the thumb-support member 116 or 216 through the longitudinal gap 120 within the syringe housing 112. Again, it should be understood that the thumb-support member 116 or 216 is coupled to the barrel 102, and, as such, the thumb-support member 116 or 216 does not actually move. Indeed, it is the syringe housing 112 that moves as it is proximally slid over the barrel 102.

The method further includes a needle tract-establishing step. The needle tract-establishing step includes establishing a needle tract from a skin surface of the patient to the fluid-containing space within the patient. Establishment of the needle tract is realized upon receiving tactile feedback resulting from a pressure differential occurring with a release of the vacuum created during the vacuum-drawing step when a tip of the needle enters the fluid-containing space.

Upon establishing the needle tract in accordance with the needle tract-establishing step, fluid such as blood from the fluid-containing space such as a blood-vessel lumen can be aspirated with the aspiration syringe 100 or 200 in a fluid-aspirating step. The fluid-aspirating step includes aspirating the fluid from the fluid-containing space with a same or different hand as the foregoing single hand over, under, or to a side of the medial portion of the aspiration syringe 100 or 200 by squeezing together the finger-support member 114 or 214 and the thumb-support member 116 or 216. Squeezing together the finger-support member 114 or 214 and the thumb-support member 116 or 216 proximally slides the syringe housing 112 over the barrel 102 and withdraws the plunger 104 from the barrel 102 creating a vacuum. The squeezing together of the finger-support member 114 or 214 and the thumb-support member 116 or 216 during the fluid-aspirating step includes moving the thumb-support member 116 or 216 through the longitudinal gap 120 within the syringe housing 112. Again, it should be understood that the thumb-support member 116 or 216 is coupled to the barrel 102, and, as such, the thumb-support member 116 or 216 does not actually move. Indeed, it is the syringe housing 112 that moves as it is proximally slid over the barrel 102.

It should be understood that while the aspiration syringes 100 and 200 are designed for three-finger aspiration and the like, some clinicians might choose to use two fingers or up to all five finger of a single, which is within the scope of the concepts provided herein.

Additional adaptations and/or modifications can appear to those of ordinary skill in the art. Accordingly, departures may be made from the particular embodiments disclosed herein.

## Claims

1. An aspiration syringe (100, 200), comprising:
a barrel (102);
a plunger (104) disposed in the barrel (102); and
an aspiration mechanism including:
a thumb-support member (116, 216) coupled to a proximal portion of the barrel (102); and
a syringe housing (112) slidably disposed around the barrel (102) including a proximal portion coupled to a proximal portion of the plunger (104) and a distal portion terminating with a flange (118) incorporated into a finger-support member (114, 214), the aspiration mechanism configured for withdrawing the plunger (104) from the barrel (102) as the finger-support member (114, 214) is slid over the barrel (102) toward the thumb-support member (116, 216).

2. The aspiration syringe of claim 1, wherein the syringe housing (112) and the plunger (104) are molded together in an integral piece such that the proximal portion of the syringe housing (112) is integral with the proximal portion of the plunger (104).

3. The aspiration syringe of claim 1, wherein the syringe housing (112) and the plunger (104) are separately molded pieces coupled together in a coupled piece such that the proximal portion of the syringe housing (112) is bonded or adhered to the proximal portion of the plunger (104).

4. The aspiration syringe of any claim of claims 1-3, wherein the syringe housing (112) includes a longitudinal gap (120) within which the thumb-support member (116, 216) moves relative to the syringe housing (112) when the syringe housing (112) is slid over the barrel (102).

5. The aspiration syringe of any claim of claims 1-4, wherein the flange (118) includes a pair of finger recesses (122) in a distal face of the flange (118) configured to respectively cradle a pair of finger pads of a pair of fingers of the single hand, the pair of finger recesses (122) configured to encourage placement of each finger of the pair of fingers on an opposite side of the barrel (102).

6. The aspiration syringe of any claim of claims 1-4, wherein the flange (118) is annular or the flange (118) is coupled to a distal annular member (224) with the flange (118) or the distal annular member (224) encircling the barrel (102), the flange (118) or the distal annular member (224) configured to accommodate a pair of finger pads of a pair of fingers of the single hand placed against a distal side thereof in any chosen placement of each finger of the pair of fingers around the flange (118) or the distal annular member (224).

7. The aspiration syringe of claim 6, wherein the flange (118) or the distal annular member (224) includes an annular finger recess in a distal face thereof, the annular finger recess configured to cradle the pair of finger pads of the pair of fingers.

8. The aspiration syringe of any claim of claims 1-7, wherein the thumb-support member (116, 216) includes a single thumb recess (126) in a proximal face of the thumb-support member (116, 216) configured to cradle a thumbpad of a thumb, the single thumb recess (126) configured to encourage approximately longitudinal alignment of the thumb with a surface normal of a surface of the barrel (102).

9. The aspiration syringe of any claim of claims 1-7, wherein the thumb-support member (116, 216) is coupled to or formed with a proximal annular member (228) with the proximal annular member (228) encircling the barrel (102), the proximal annular member (228) configured to accommodate a thumbpad of a thumb placed against a proximal side thereof in any chosen placement of the thumb around the proximal annular member (228).

10. The aspiration syringe of claim 9, wherein the proximal annular member (228) includes an annular thumb recess in a proximal face thereof, the annular thumb recess configured to cradle the thumbpad of the thumb.

11. The aspiration syringe of any claim of claims 1-10, wherein a distal portion of the barrel (102) terminates with a Luer-tapered syringe tip (106).

12. The aspiration syringe of claim 11, wherein the distal portion of the barrel (102) further terminates with an internally threaded connector (108) around the syringe tip (106) configured to screw together with an externally threaded or flanged connector of another medical device.

## Patentansprüche

1. Aspirationsspritze (100, 200), umfassend:
einen Zylinder (102);
eine Kolbenstange (104), die im Zylinder (102) angeordnet ist; und
einen Aspirationsmechanismus, einschließend:
ein Daumenstützelement (116, 216), das mit einem proximalen Abschnitt des Zylinders (102) gekoppelt ist; und
ein Spritzengehäuse (112), das verschiebbar um den Zylinder (102) herum angeordnet ist, einschließend einen proximalen Abschnitt, der mit einem proximalen Abschnitt der Kolbenstange (104) gekoppelt ist, und einen distalen Abschnitt, der mit einem Flansch (118) endet, der in ein Fingerstützelement (114, 214) eingearbeitet ist, wobei der Aspirationsmechanismus zum Herausziehen der Kolbenstange (104) aus dem Zylinder (102) konfiguriert ist, wenn das Fingerstützelement (114, 214) in Richtung zu dem Daumenstützelement (116, 216) über den Zylinder (102) geschoben wird.

2. Aspirationsspritze nach Anspruch 1, wobei das Spritzengehäuse (112) und die Kolbenstange (104) zusammen in einem integralen Teil geformt sind, sodass der proximale Abschnitt des Spritzengehäuses (112) mit dem proximalen Abschnitt der Kolbenstange (104) integral ist.

3. Aspirationsspritze nach Anspruch 1, wobei das Spritzengehäuse (112) und die Kolbenstange (104) separat geformte Teile sind, die in einem gekoppelten Teil zusammengekoppelt sind, sodass der proximale Abschnitt des Spritzengehäuses (112) mit dem proximalen Abschnitt der Kolbenstange (104) verbunden oder verklebt ist.

4. Aspirationsspritze nach einem der Ansprüche 1-3, wobei das Spritzengehäuse (112) einen Längsspalt (120) einschließt, innerhalb dessen sich das Daumenstützelement (116, 216) relativ zum Spritzengehäuse (112) bewegt, wenn das Spritzengehäuse (112) über den Zylinder (102) geschoben wird.

5. Aspirationsspritze nach einem der Ansprüche 1-4, wobei der Flansch (118) ein Paar von Fingeraussparungen (122) in einer distalen Stirnfläche des Flansches (118) einschließt, die konfiguriert sind, um jeweils ein Paar von Fingerballen eines Paares von Fingern der einzigen Hand zu halten, wobei das Paar von Fingeraussparungen (122) konfiguriert ist, um die Platzierung jedes Fingers des Paares von Fingern auf einer gegenüberliegenden Seite des Zylinders (102) anzuregen.

6. Aspirationsspritze nach einem der Ansprüche 1-4, wobei der Flansch (118) ringförmig ist oder der Flansch (118) mit einem distalen ringförmigen Element (224) gekoppelt ist, wobei der Flansch (118) oder das distale ringförmige Element (224) den Zylinder (102) umgibt, wobei der Flansch (118) oder das distale ringförmige Element (224) konfiguriert ist, um ein Paar von Fingerballen eines Paares von Fingern der einzigen Hand aufzunehmen, die bei einer beliebigen gewählten Platzierung jedes Fingers des Paares von Fingern an einer distalen Seite davon um den Flansch (118) oder das distale ringförmige Element (224) herum platziert ist.

7. Aspirationsspritze nach Anspruch 6, wobei der Flansch (118) oder das distale ringförmige Element (224) eine ringförmige Fingeraussparung in einer distalen Stirnfläche davon einschließt, wobei die ringförmige Fingeraussparung konfiguriert ist, um das Paar von Fingerballen des Paares von Fingern zu halten.

8. Aspirationsspritze nach einem der Ansprüche 1-7, wobei das Daumenstützelement (116, 216) eine einzige Daumenaussparung (126) in einer proximalen Stirnfläche des Daumenstützelements (116, 216) einschließt, die konfiguriert ist, um einen Daumenballen eines Daumens zu halten, wobei die einzige Daumenaussparung (126) konfiguriert ist, um eine ungefähre Längsausrichtung des Daumens mit einer Oberflächennormalen einer Oberfläche des Zylinders (102) anzuregen.

9. Aspirationsspritze nach einem der Ansprüche 1-7, wobei das Daumenstützelement (116, 216) mit einem proximalen ringförmigen Element (228) gekoppelt oder mit diesem gebildet ist, wobei das proximale ringförmige Element (228) den Zylinder (102) umgibt, wobei das proximale ringförmige Element (228) konfiguriert ist, um einen Daumenballen eines Daumens aufzunehmen, der bei einer beliebigen gewählten Platzierung des Daumens an einer proximalen Seite davon um das proximale ringförmige Element (228) herum platziert ist.

10. Aspirationsspritze nach Anspruch 9, wobei das distale ringförmige Element (228) eine ringförmige Daumenaussparung in einer proximalen Stirnfläche davon einschließt, wobei die ringförmige Daumenaussparung konfiguriert ist, um den Daumenballen des Daumens zu halten.

11. Aspirationsspritze nach einem der Ansprüche 1-10, wobei ein distaler Abschnitt des Zylinders (102) mit einer Luer-konischen Spritzenspitze (106) endet.

12. Aspirationsspritze nach Anspruch 11, wobei der distale Abschnitt des Zylinders (102) weiter mit einem Innengewindeanschluss (108) um die Spritzenspitze (106) herum endet, der konfiguriert ist, um mit einem Außengewindeanschluss oder einem angeflanschten Anschluss einer anderen medizinischen Vorrichtung verschraubt zu werden.

## Revendications

1. Seringue d'aspiration (100, 200), comprenant :
un corps (102) ;
un piston (104) disposé dans le corps (102) ; et
un mécanisme d'aspiration incluant :
un élément de support de pouce (116, 216) accouplé à une partie proximale du corps (102) ; et
un boîtier de seringue (112) disposé de manière coulissante autour du corps (102) incluant une partie proximale accouplée à une partie proximale du piston (104) et une partie distale se terminant par une bride (118) incorporée dans un élément de support de doigt (114, 214), le mécanisme d'aspiration étant configuré pour retirer le piston (104) du corps (102) lorsque l'on fait glisser l'élément de support de doigt (114, 214) sur le corps (102) vers l'élément de support de pouce (116, 216).

2. Seringue d'aspiration selon la revendication 1, dans laquelle le boîtier de seringue (112) et le piston (104) sont moulés ensemble en une seule pièce de sorte que la partie proximale du boîtier de seringue (112) fait partie intégrante de la partie proximale du piston (104).

3. Seringue d'aspiration selon la revendication 1, dans laquelle le boîtier de seringue (112) et le piston (104) sont des pièces moulées séparément accouplées ensemble en une pièce accouplée de sorte que la partie proximale du boîtier de seringue (112) est liée ou collée à la partie proximale du piston (104).

4. Seringue d'aspiration selon l'une quelconque des revendications 1 à 3, dans laquelle le boîtier de seringue (112) inclut un espace longitudinal (120) à l'intérieur duquel l'élément de support de pouce (116, 216) se déplace par rapport au boîtier de seringue (112) lorsque l'on fait glisser le boîtier de seringue (112) sur le corps (102).

5. Seringue d'aspiration selon l'une quelconque des revendications 1 à 4, dans laquelle la bride (118) inclut une paire d'évidements pour doigts (122) dans une face distale de la bride (118) configurée pour accueillir respectivement une paire de coussinets de doigts d'une paire de doigts d'une seule main, la paire d'évidements pour doigts (122) étant configurée pour encourager le placement de chaque doigt de la paire de doigts sur un côté opposé du corps (102).

6. Seringue d'aspiration selon l'une quelconque des revendications 1 à 4, dans laquelle la bride (118) est annulaire ou la bride (118) est couplée à un élément annulaire distal (224) avec la bride (118) ou l'élément annulaire distal (224) encerclant le corps (102), la bride (118) ou l'élément annulaire distal (224) sont configurés pour recevoir une paire de coussinets de doigts d'une paire de doigts de la main unique placés contre un côté distal de celle-ci ou de celui-ci dans n'importe quel placement choisi de chaque doigt de la paire de doigts autour de la bride (118) ou de l'élément annulaire distal (224).

7. Seringue d'aspiration selon la revendication 6, dans laquelle la bride (118) ou l'élément annulaire distal (224) inclut un évidement annulaire pour doigt dans une face distale de celle-ci ou de celui-ci, l'évidement annulaire pour doigt étant configuré pour accueillir la paire de coussinets de doigt de la paire de doigts.

8. Seringue d'aspiration selon l'une quelconque des revendications 1 à 7, dans laquelle l'élément de support de pouce (116, 216) inclut un évidement unique pour le pouce (126) dans une face proximale de l'élément de support de pouce (116, 216) configuré pour accueillir un coussinet de pouce d'un pouce, l'évidement unique pour le pouce (126) étant configuré pour encourager un alignement approximativement longitudinal du pouce avec une surface normale à une surface du corps (102).

9. Seringue d'aspiration selon l'une quelconque des revendications 1 à 7, dans laquelle l'élément de support de pouce (116, 216) est couplé à ou formé avec un élément annulaire proximal (228), l'élément annulaire proximal (228) encerclant le corps (102), l'élément annulaire proximal (228) étant configuré pour recevoir un coussinet de pouce d'un pouce placé contre un côté proximal de celui-ci dans n'importe quel placement choisi du pouce autour de l'élément annulaire proximal (228).

10. Seringue d'aspiration selon la revendication 9, dans laquelle l'élément annulaire proximal (228) inclut un évidement annulaire pour le pouce dans une face proximale de celui-ci, l'évidement annulaire pour le pouce étant configuré pour accueillir le coussinet de pouce du pouce.

11. Seringue d'aspiration selon l'une quelconque des revendications 1 à 10, dans laquelle une partie distale du corps (102) se termine par un embout de seringue (106) conique Luer.

12. Seringue d'aspiration selon la revendication 11, dans laquelle la partie distale du corps (102) se termine en outre par un raccord fileté intérieurement (108) autour de l'embout de seringue (106) configuré pour se visser avec un raccord fileté extérieurement ou à bride d'un autre dispositif médical.
